# EUROPEAN PATENT APPLICATION

(11) **EP 4 431 945 A1**
(43) Date of publication of application: **18.09.2024**
(21) Application number: 23382248.5
(22) Date of filing: 16.03.2023
(51) Int. Cl.: G01N 33/92, G01N 33/68

(54) **BIOMARKERS DISCRIMINATING THE DISCORDANT PHENOTYPES OF X-ADRENOLEUKODYSTROPHY**

(71) Applicant: Institut d'Investigació Biomèdica de Bellvitge (IDIBELL), 08907 Hospitalet de Llobregat, Barcelona (ES); Institució Catalana de Recerca I Estudis Avançats, 08010 Barcelona (ES); Consorcio Centro de Investigación Biomédica en Red, 28029 Madrid (ES); Asociación Española Contra la Leucodistrofia ELA España, 28823 Madrid (ES)
(72) Inventor: PUJOL ONOFRE, Aurora, 08907 Barcelona (ES); SCHLÜTER MARTIN, Agatha, 08907 Barcelona (ES); FOURCADE, Stéphane, 08907 Barcelona (ES); RUIZ SALES, Montserrat, 08907 Barcelona (ES); PLANAS SERRA, Laura, 08907 Barcelona (ES); CASANOVAS PONS, Carlos, 08907 Barcelona (ES)
(74) Representative: Hoffmann Eitle

(57) **Abstract**

The present invention provides lipid markers highly precise in the diagnosis, prognosis, and stratification of subjects suffering from X-ALD.

These markers allow predicting or monitoring the response or efficacy to a particular medical regimen.

## Description

### FIELD OF THE INVENTION

The present invention is related to diagnostic field, in particular with the precise stratification of X-linked adrenoleukodystrophy (X-ALD) using lipidic biomarkers, as well as the prediction/determination to a particular medical regimen.

### BACKGROUND ART

X-linked adrenoleukodystrophy (X-ALD) (OMIM 300100) is the most frequent peroxisomal disorder with a minimum incidence of 1:14,700 births. It is a severe neurometabolic disease characterized by progressive demyelination in the central nervous system (CNS), adrenal insufficiency, and accumulation of very long-chain fatty acids (VLCFA, mainly C26:0). The molecular cause is the loss of function of the ABCD1 transporter which imports these VLCFA for degradation into the peroxisomes. The exact molecular mechanisms of toxicity of these fatty acids in the brain are unknown, although it has been reported that excess of VLCFA provokes mitochondrial transport chain dysfunction and free radicals generation, with failed endogenous antioxidant response, proteostasis and increased ER-stress response.

The main phenotypes of X-ALD are: cerebral ALD (cALD) including childhood cerebral (ccALD), adolescent cerebral (AdoICALD), adult cerebral (acALD); and adrenomyeloneuropathy (AMN).

Childhood cerebral ALD or ccALD (35% of cases), which affects children between 5-12 years of age and is associated with inflammatory demyelination, with rapid and fatal progression in a few years

Adrenomyeloneuropathy (AMN), a form that affects the corticospinal tracts in the spinal cord and peripheral nerves in adult men between 20-50 years. 30-50% of AMN patients also develop the cerebral inflammatory form at some time (cerebral AMN; cAMN).

This broad spectrum of clinical variation occurs in the same family nucleus, suggesting the involvement of genetic, stochastic, or epigenetic modifiers unknown to date. Indeed, the phenotype cannot be predicted by the nature of the ABCD1 mutation, as the same mutation can be associated with each of the known phenotypes. Mild phenotypes may be associated with large deletions that abolish formation of the gene product, and severe phenotypes occur with missense pathogenic variants in which abundant immunoreactive protein product is produced. Therefore, the various clinical types of X-ALD can be observed in the same kindreds and nuclear families carrying the same mutation in the ABCD1 gene. Measurement of plasma VLCFA levels, particularly C26:0 VLCFA, is the standard test for X-ALD diagnosis; however, the levels of C26:0 in plasma are not correlated with severity of disease or phenotype. Most fatty acyl chains are not present in their free form in biological samples but are incorporated into complex lipids such as phospholipids (PLs) and sphingolipids (SL), which are main components of cellular membranes and the myelin sheath. Several inherited disorders of sphingolipid (SL) metabolism include disruptions of myelin sheath formation and maintenance, thus the investigation of complex lipid species may provide clues to discriminative biomarkers for X-ALD divergent phenotypes.

For AMN, which represents 85% of X-ALD cases (males and females combined), no curative therapy is available; however, therapeutic options are currently under investigation. Allogenic bone marrow transplantation is associated with high morbidity and mortality and it is recommended only in nearly asymptomatic ccALD and cAMN in a very narrow therapeutic window. The hematopoietic stem cell gene therapy with lentiviral vector correcting CD34⁺ stem cells is a good alternative to transplantation in children without compatible donors. In practice, most patients with ccALD are diagnosed too late to be treated, which is why neonatal screening based on VLCFA levels was approved in the USA and the Netherlands, not without controversy due to the lack of discriminatory markers between phenotypes.

Thus, the identification and validation of biomarkers able to discriminate across the phenotypic spectrum is an unmet, urgent need in X-ALD population.

### SUMMARY OF THE INVENTION

The inventors have surprisingly found, for the first time, that there were discriminating lipids among X-ALD phenotypes when plasma and brain white matter samples were analysed and compared.

As it is explained below, the inventors used plasma (ccALD and AMN) and normal-appearing brain white matter (NAWM) from (ccALD and cAMN) patients, and age-matched healthy individuals and applied lipidomic technology to find differences among the discordant disease phenotypes. Surprisingly, these lipidomic experiments in NAWM revealed that several complex lipids containing VLCFA or belonging to lipid droplets were increased in cerebral X-ALD patients.

Remarkably, the sphingolipid (SL) galactosylceramide (GalCer) is reduced in ccALD patients compared to cAMN which correlates to the down-regulation of *UGT8,* the enzyme encoding for the ceramide galactosyltransferase (CGT) which converts ceramide (Cer) to GalCer. A total of 66 lipids in plasma and 18 lipids in brain white matter were found to be differentially detected (as shown in Tables 1 and 2, respectively).

The identification of so sensitive lipidic biomarkers, using routinary means for determining the presence/amount of lipids, represents a great advance in the appropriate identification of X-ALD phenotype and, consequently, in the therapeutic management of this disease because these biomarkers will allow:
1) To monitor response to treatment in clinical trials of novel compounds or biologics; and
2) To inform on disease severity and prognosis to define the personalized management of each patient.

Thus, in a first aspect the present invention provides an *in vitro* method for classifying a subject suffering from an X-linked adrenoleukodystrophy as suffering from the cerebral inflammatory adrenoleukodystrophy (cALD) phenotype or as suffering from the adrenomyeloneuropathy (AMN) phenotype, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
(b) obtaining a reference value for each one of the lipidic components in a population already identified as having cALD phenotype or an AMN phenotype;
(c) comparing each one of the lipidic components determined in step (a) with the corresponding reference value; and
(d) identifying a deviation of the lipid level in the test sample with respect to the cALD orAMN reference value.

It is of special relevance the fact that it was found that the lipids correlating with ccALD and cAMN were differentially found in very early stages. In fact, it was found that they were significantly altered at a stage wherein the white matter was still intact. This is of special relevance because as it has been discussed above, one of the main obstacles in the managing of this disease is that it is silent in the first stages, so that when it is finally diagnosed it is so advance that there is no efficient therapy in the most cases.

Thus, in a second aspect the present invention provides an *in vitro* method of diagnosis cALD, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
(b) obtaining a reference value for each one of the lipidic components in a population already identified as having an AMN phenotype;
(c) comparing each one of the quantified lipidic components determined in step (a) with the corresponding reference value; and
(d) identifying a deviation of the lipid level from the test sample with respect to the AMN reference value,
wherein the subject will suffer from cALD phenotype when the isolated test sample comprises one or more of the markers of Table 3 at a level above the corresponding reference value(s); and/or one or more markers of Table 4 below the corresponding reference value(s).

In a third aspect the present invention provides an *in vitro* method of diagnosis or prognosis of AMN, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
(b) obtaining a reference value for each one of the lipidic components in a population already identified as having an AMN phenotype;
(c) comparing each one of the quantified lipidic components determined in step (a) with the corresponding reference value; and
(d) identifying a deviation of the lipid level from the test sample with respect to the AMN reference value,
wherein the subject will have an AMN phenotype when the isolated test sample comprises one or more of the markers of Table 3 or the cholic acid is at a level equal or lower than the corresponding reference value(s); and/or one or more markers of Table 4 above the corresponding reference value(s).

In a fourth aspect the present invention provides an *in vitro* method for determining the response of a treatment in a subject suffering from cALD, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3; in two samples of the subject, one of them taken before and the other taken after starting the therapy; or, alternatively, each one of them taken in two separate moments after starting the therapy; and
(b) comparing the quantified lipidic components in the two moments of time;
wherein
when the level of one or more of the markers of Table 3 is reduced; and/or the level of one or more markers of Table 4 is increased, it will be indicative that the subject positively responds to the treatment.

In a fifth aspect the present invention provides an *in vitro* method for determining the response of a treatment in a subject suffering from AMN, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3; in two samples of the subject, one of them taken before and the other taken after starting the therapy; or, alternatively, each one of them taken in two separate moments after starting the therapy; and
(b) comparing the quantified lipidic components in the two moments of time;
wherein
when the level of one or more of the markers of Table 3 is increased; and/or the level of one or more markers of Table 4 is decreased, it is indicative that the subject positively responds to the treatment.

The present inventors have also found that the level of some ceramide synthase, but specially of UGT-8, is remarkably down-expressed in patients suffering from ccALD.

Thus, in a further aspect the present invention provides an *in vitro* diagnostic method of cALD in a subject, the method comprising:
- determining the gene expression level of one of the following enzymes: ceramide synthase (CerS) 1, 4, 6, GAL3ST1, FA2H, or the UGT-8, and
- comparing with the corresponding reference value from a healthy children population;
wherein when the gene expression level is lower than the reference value, this will indicate that the subject has an increased risk to suffer from cALD.

In a final aspect, the present invention provides a screening method for identifying candidate compounds suitable for the treatment of cALD, the method comprising contacting the candidate compound with a medium comprising one of the following enzymes: ceramide synthase (CerS) 1, 4, 6, GAL3ST1, FA2H or the UGT-8, and determining the gene expression level, provided that when the compound increases the gene expression level, this will mean that the tested compound is a candidate for the treatment of cALD.

### BRIEF DESCRIPTION OF DRAWINGS

**Figure 1****. Heatmap representation of the differential metabolites in ccALD respect to AMN.** Heatmap representation of peak intensities and log₂-fold changes of the differential metabolites in ccALD respect to AMN adjusted by age (p adjusted < 0.05). Each line represents a significant lipid species colored according to its abundance intensity normalized to the median across the samples. The scale from blue to red represents this normalized abundance in arbitrary units.
**Figure 2****.** Lipidomic profiles in plasma from control and X-ALD subjects (n = 20 adult X-ALD, n = 14 ccALD, n = 15 Adult controls and n = 15 child controls). Correlation between SM (d18:2/22:1) and GD1 (d18:1/ 20:0) levels in X-ALD adults, ccALD, adult and child controls.
**Figure 3****. Lipidomic profiles in the non-affected white brain from X-ALD patients.** Heatmap representation of peak intensities and log2 fold changes of the differential metabolites in ccALD respect to cAMN adjusted by age (p < 0.05). Each line represents a significant lipid species.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present invention provides new biomarkers for the stratification, diagnosis or prognosis of subjects suffering from X-ALD.

The term "diagnosis" is known to the person skilled in the art. As used herein "diagnosis" is understood as becoming aware of a particular medical condition complication or risk in a subject; the determination of the nature of the disease or condition; or the distinguishing of one disease or condition from another. It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification.

"Prognosis" as used herein refers to the prediction of the probable progression and outcome of a disease.

In the present invention, the term "reference value " referred to in the methods of the invention is to be understood as a predefined value of a given molecular marker, in the present case any of the markers pointed out in any of the aspects and embodiments, which is derived from the levels of said molecular marker in a sample or group of samples. If the level of expression is determined at the protein level (as it is the case of interleukines), then the "reference expression level" is a predefined value of protein quantity, whereas if the level of expression is determined at the mRNA level, then the "reference expression level" is a predefined value of mRNA quantity. This value is used as a threshold to discriminate subjects wherein the condition to be analyzed is present from those wherein such condition is absent to determine the stage of the disease, the risk of developing or of being suffering cALD, cAMN, or AMN , among others. This reference value is also useful for determining whether the subject has to initiate a medical regimen and how effective the regimen is.

Methods for obtaining the reference value from the group of subjects selected are well-known in the state of the art (Burtis C. A. et al., 2008, Chapter 14, section "Statistical Treatment of Reference Values") In a particular case "reference value" is a cut-off value defined by means of a conventional ROC analysis (Receiver Operating Characteristic analysis). As the skilled person will appreciate, optimal cut-off value will be defined according to the particular applications of the diagnostic or prognostic method: purpose, target population for the diagnosis or prognosis, balance between specificity and sensibility, etc.

Collecting information on a lipidomic marker (i.e., the concentration(s) of (a) lipid(s), lipid-lipid ratio(s)) from the sample of a patient and, where appropriate, a corresponding control sample, can be performed with various chemical and high-resolution analytical techniques. Suitable analytical techniques include, but are not limited to, mass spectrometry and nuclear resonance spectroscopy. Any high-resolution technique capable of resolving individual lipids or lipid classes and providing structural information of the same can be used to collect the information on the lipidomic marker in question, e.g., lipid profile from the biological sample. Collecting the information on the lipidomic marker with mass spectrometry (MS) is one of the preferred embodiments of the current invention. The MS instrument can be coupled to a direct sample infusion method, such as a robotic nanoflow ion source device, or to a high performance separation method such as high performance liquid chromatography (HPLC) or ultra performance liquid chromatography (UPLC).

In an embodiment of the methods of the invention, the results can be analyzed by at least one algorithm. In some embodiments, the at least one algorithm can be selected from the group of: a cluster analysis algorithm, a cumulative quantification algorithm, and any combination thereof, and a cluster analysis.

Again, in accordance with all aspects and embodiments described and claimed herein, both the sample from the subject and the control sample is preferably a blood sample, more preferably a blood plasma sample, or also preferably a blood serum sample. It may also be a fraction of blood, blood plasma or blood serum, e.g., a lipoprotein fraction. A blood sample can be prepared and plasma or serum, or fractions thereof, can be separated therefrom with techniques well known to the person skilled in the art.

Alternatively, the sample can be cerebrospinal fluid. As it is shown below, the markers forming part of the invention can be differentially found, depending on the particular phenotype of the subject, in the white matter of the brain. As the skilled person will recognize, the differential behaviour of the lipids in the white matter can also be extrapolated to what it could be found on the cerebrospinal liquid, which is irrigating the white matter.

In the context of the invention, when in the first aspect of the invention reference is made to a "have an AMN phenotype" will encompass the mild (AMN) and severe (cAMN) phenotypes.

In an embodiment of the first aspect of the invention, the reference value for each one of the lipids is obtained from a population of subjects with an AMN phenotype, and:
the subject will have cALD phenotype when the isolated test sample comprises one or more of the markers of Table 3 at a level above the corresponding reference value(s); and/or one or more markers of Table 4 below the corresponding reference value(s); and the subject will have an AMN phenotype when the isolated test sample comprises one or more of the markers of Table 3 at a level equal or lower than the corresponding reference value(s); and/or one or more markers of Table 4 above the corresponding reference value(s):

**Table 3. Lipid levels significantly higher in ccALD with respect to AMN or cAMN in plasma and brain of XALD patients. A two-factor analysis to adjust age effect between XALD phenotypes by including two-levels, children and adult in the specification of the Age factor. For oxylipids a linear mixed model was computed with age as a covariate and a random patient ID factor to account for patient replications. Cholesteryl esters (CE), ceramides (Cer), diacylglycerols (DAG), dihexosylceramides (DiHexCer), free fatty acids (FFA), fatty acylcarnitines (AC), hexosylceramides (HexCer), lysophosphatidylcholines (LPC), lysophosphatidylethanolamines (LPE), phosphatidylcholines, (PC), phosphatidylethanolamines (PE), oxylipids (OL), phosphatidylglycerols (PG), phosphatidylinositols (PI), phosphatidylserines (PS), sphingosines (SPH), and sphingomyelins (SM), and triacylglycerols (TAG).**

| | | ccALDvsAMN | ccALDvscAMN | |
|---|---|---|---|---|
| productNameShort | class | P.Value | P.Value | tissue |
| Cer (d18:0/C19:Q(OH)) | Cer | | 0.0328 | Brain |
| Cer (dl8:0/C20:0(OH)) | Cer | | 0.0051 | Brain |
| Cer (d18:0/C22:0(OH)} | Cer | | 0.0046 | Brain |
| Cer (d18:0/C24:0(OH)) | Cer | | 0.0067 | Brain |
| 18:3 Cholesteryl ester | CE | 0.0175 | | plasma |
| 20:3 Cholesteryl ester | CE | 0.0167 | | plasma |
| 22:1 Cholesteryl ester | CE | | 0.0336 | Brain |
| DG 36a:5 | DAG | 0.0115 | | plasma |
| FA C16:0 (OH) | FFA | | 0.0132 | Brain |
| FA C22:1 | FFA | 0.0433 | | plasma |
| 11-HETE | OL | 0.0266 | | plasma |
| 11,12-DHET | OL | 0.0258 | | plasma |
| 15-oxoETE | OL | 0.0216 | | plasma |
| 17-HDHA | OL | | | plasma |
| 5-HETE | OL | 0.0102 | | plasma |
| 5,6-DHET | OL | 0.0035 | | plasma |
| 6R-LXA4 | OL | 0.0056 | | plasma |
| 8,9-DHET | OL | 0.0072 | | plasma |
| 9-HETE | OL | 0.0187 | | plasma |
| PC 30a:1 | PC | 0.0421 | | plasma |
| PC 31a:1 | PC | 0.0105 | | plasma |
| PC 31a:1 | PC | 0.0433 | | plasma |
| PC 32a:1 | PC | 0.0355 | | plasma |
| PC 32p:0 | PC | | 0.0273 | Brain |
| PC 33a:1 | PC | 0.0231 | | plasma |
| PC 33a:4 | PC | 0.0483 | | plasma |
| PC 34a:3 | PC | 0.0217 | | plasma |
| PC 34a:4 | PC | 0.0485 | | plasma |
| PE 34a:1 | PE | 0.0175 | | plasma |
| PE 36a:1 | PE | 0.0421 | | plasma |
| PE 38p:4 | PE | | 0.0382 | Brain |
| Sphingosine d18:1 | SPH | | 0.0479 | Brain |
| PG 34a:2 | PG | 0.0230 | | plasma |
| PI 34a:3 | PI | 0.0042 | | plasma |
| PI 36a:5 | PI | 0.0299 | | plasma |
| PI 38a:5 | PI | 0.0205 | | plasma |
| TG 54:6 | TAG | 0.0217 | | plasma |
| TG 58:2 | TAG | 0.0273 | | plasma |
| TG 58:3 | TAG | 0.0088 | | plasma |
| TG 58:4 | TAG | 0.0176 | | plasma |
| TG 60:3 | TAG | 0.0453 | | plasma |
| TG 60:4 | TAG | 0.0155 | | plasma |
| TG 60:5 | TAG | 0.0088 | | plasma |
| TG 62:3 | TAG | 0.0323 | | plasma |
| TG 66:3 | TAG | 0.0421 | | plasma |

**Table 4. Lipids with levels significantly lower in ccALD with respect to AMN or cAMN in plasma and brain samples of XALD patients. A two-factor analysis was performed to adjust age effect between XALD phenotypes by including two-levels, children and adult in the specification of the Age factor. For oxylipids a linear mixed model was computed with age as a covariate and a random patient ID factor to account for patient replications. Cholesteryl esters (CE), ceramides (Cer), diacylglycerols (DAG), dihexosylceramides (DiHexCer), free fatty acids (FFA), fatty acylcarnitines (AC), hexosylceramides (HexCer), lysophosphatidylcholines (LPC), lysophosphatidylethanolamines (LPE), phosphatidylcholines, (PC), phosphatidylethanolamines (PE), oxylipids (OL), phosphatidylglycerols (PG), phosphatidylinositols (PI), phosphatidylserines (PS), sphingosines (SPH), and sphingomyelins (SM), and triacylglycerols (TAG).**

| | | ccALDvsAMN | ccALDvscAMN | |
|---|---|---|---|---|
| productNameShort | class | P.Value | P.Value | Tissue |
| Cholic acid | BA | | | plasma |
| 16:0 Cholesteryl ester | CE | 0.0114 | | plasma |
| 18:2 Cholesteryl ester | CE | 0.0202 | | plasma |
| 20:1 Cholesteryl ester | CE | 0.0131 | | plasma |
| DG 33a:4 | DAG | 0.0176 | | plasma |
| DG 36a:0 | DAG | 0.0074 | | plasma |
| DG 42a:6 | DAG | 0.0268 | | plasma |
| DiHexCer (dl8:0/C20:0(OH)) | DiHexCer | | 0.0387 | brain |
| FA C16:0 (OH) | FFA | 0.0485 | | plasma |
| FA C18:0 (OH) | FFA | 0.0485 | | plasma |
| C16:0 Carnitine | AC | 0.0175 | | plasma |
| C18:1 Carnitine | AC | 0.0260 | | plasma |
| C18:2 Carnitine | AC | 0.0386 | | plasma |
| C20:4 Carnitine | AC | 0.0120 | | plasma |
| HexCer (d18:1/C23:1(OH)) | HexCer | | 0.0428 | brain |
| HexCer (d18:2/C24:1(OH)) | HexCer | | 0.0432 | brain |
| HexCer (d18:2/C25:1(OH)) | HexCer | | 0.0322 | brain |
| LPC 13a:0 | LPC | 0.0283 | | plasma |
| LPC 16a:0 | LPC | 0.0175 | | plasma |
| LPC 16e:0 | LPC | 0.0020 | | plasma |
| LPC 16p:0 | LPC | 0.0020 | | plasma |
| LPC 16p:1 | LPC | 0.0169 | | plasma |
| LPC 18a:0 | LPC | 0.0299 | | plasma |
| LPC 18p:0 | LPC | 0.0020 | | plasma |
| LPC 19a:3 | LPC | 0.0386 | | plasma |
| LPC 21a:4 | LPC | | 0.0301 | brain |
| LPE 18a:0 | LPE | 0.0169 | | plasma |
| LPE 20a:0 | LPE | 0.0386 | | plasma |
| PC 30a:3 | PC | 0.0088 | | plasma |
| PC 33a:4 | PC | 0.0155 | | plasma |
| PC 37p:6 | PC | 0.0386 | | plasma |
| PC 37p:7 | PC | 0.0355 | | plasma |
| PC 38a:1 | PC | | 0.0339 | brain |
| PC 38p:4 | PC | 0.0433 | | plasma |
| PC 38p:5 | PC | 0.0175 | | plasma |
| PC 38p:6 | PC | 0.0130 | | plasma |
| PC 40p:5 | PC | | 0.0380 | brain |
| PC 40p:6 | PC | 0.0119 | | plasma |
| PC 42a:5 | PC | | 0.0440 | brain |
| PC 42p:3 | PC | | 0.0244 | brain |
| PE 28a:0 | PE | 0.0387 | | plasma |
| PE 37a:7 | PE | 0.0433 | | plasma |
| PE 38p:6 | PE | 0.0231 | | plasma |
| PE 40p:6 | PE | 0.0175 | | plasma |
| PE 40p:7 | PE | 0.0074 | | plasma |
| PG 40p:6 | PG | 0.0072 | | plasma |
| PI 40a:3 | PI | 0.0404 | | plasma |
| PI 40a:4 | PI | 0.0176 | | plasma |
| PS 29e:0 | PS | 0.0105 | | plasma |
| PS 39p:0 | PS | 0.0072 | | plasma |
| SM (d18:1/C16:1) | SM | 0.0273 | | plasma |

In an embodiment of the first aspect of the invention, step (b) further comprises determining the level of each one of the lipids in a population of samples from healthy people, both adults and children, and step (c) is performed by a two-factor statistical analysis.

In a further embodiment of the first aspect of the invention, step (b) comprises determining the level of one or more of GD1, SM, LPC 26:0, LPC 26:1, and GM3.

In a further embodiment of the first aspect of the invention, it is determined the level of GD1, SM and LPC 26:1; or alternatively of GD1, SM, and LPC 26:0.

In a further aspect the present invention provides an *in vitro* method of diagnosis or prognosis of AMN. In one embodiment, this method is a prognostic method, wherein when one or more of the following oxylipids: 11-HETE, 11,12-DHET, 15-oxoETE, 17-HDHA, 5-HETE, 5,6-DHET, 6R-LXA4, 8,9-DHET, and 9-HETE, is higher than the corresponding reference value(s), this will be indicative of bad prognosis. In one embodiment, the "bad prognosis" is the evolution to a cerebral inflammatory form (cAMN).

In one embodiment of any of the aspects of the invention, it is determined one or more of the following markers:
- the combination of GD1, SM and LPC 26:1;
- oxylipids listed in Table 3; and
- cholic acid in Table 4;

in an isolated plasma sample of the subject.

In another embodiment of any of the aspects of the invention, it is determined the level of one or more galactosylceramides in an isolated cerebrospinal fluid sample of the subject.

In any of the embodiments of the invention, it is determined the level of expression of one or more of IL-18, IL-1RA or UGT8.

In the context of the invention, the term "level of expression" encompasses both the determination of the mRNA level or the enzymatic activity. There are currently available tools and commercial kits to determine the level as mRNA (see examples below) or by determining the particular activity in an isolated sample.

In the context of the invention, the expression "determining the response" to a particular treatment also encompasses predicting the response to the particular therapy. And, in the context of the invention the "response" to a particular therapy can also be understood as the efficacy in improving or ameliorating one or more of the symptoms related to the disease.

To those skilled in the art, other objects, advantages or features of the invention will be apparent in part from the description or in part from the practice of the invention. The following examples are provided by way of illustration or are not intended to be limiting of the present invention.

### EXAMPLES

### MATERIALS AND METHODS

### Blood sampling

Peripheral venous blood samples from X-ALD patients (diagnosed as ccALD, AMN or cAMN) and healthy controls (both children and adults) referring to Bellvitge University Hospital (Spain) were collected in standard EDTA containing tubes. 4 ml of blood were gently added to 4 ml of Histopaque (Sigma-Aldrich) and centrifuged at 400xg for 30 min at room temperature without brake. Plasma, the uppermost layer, was immediately collected and transferred with a sterile Pasteur pipette into a cryovial. Samples were directly frozen at -80°C. The use of all samples was approved by the Clinical Research Ethics Committee of the Bellvitge University Hospital (PR202/17). Informed written consent was obtained from all patients and control individuals.

### Human brain samples

Brain tissue samples from X-ALD patients (ccALD, and cAMN) and age-matched controls (healthy adults and children) were obtained from the National Institutes of Health (NIH) NeuroBioBank. Frozen blocks of normal-appearing white matter (NAWM) were dissected from the frontal or parietal lobes of controls and ccALD and cAMN patients. All children and adults with cerebral ALD had the most common parieto-occipital form of cerebral ALD. White matter sections from X-ALD patients and controls were stained with luxol fast blue (LFB) to detect demyelination, demyelination edges, and normal-looking areas. Brain tissue sections were processed when two to three adjacent sections showed no sign of demyelination by LFB staining and no perivascular cuffs of lymphocytes by haematoxylin and eosin staining

### Lipidomic analysis

Lipidomic analyses of the human brain non-affected white matter were performed according to the modified Folch method (1). For each sample, the solvent volume was adjusted to the weight of the fresh tissue. In brief, 190 µL of CHCl₃/MeOH 2:1 (v/v) and 10 µL of internal standard mixture were added to 10 mg of spinal cord. The samples were vortexed for 60 s and then sonicated for 30 s using a sonication probe. Extraction was performed after incubation for 2 h at 4°C and centrifugation at 15,000×g for 10 min at 4°C. The upper phase (aqueous phase), which contained ganglioside species and several lysophospholipids, was transferred and dried under a stream of nitrogen. The protein interphase was discarded, and the lower lipid-rich phase (organic phase) was pooled with the dried upper phase. The samples were then reconstituted in 200 µl of CHCl₃/MeOH 2:1, vortexed for 30 s, and sonicated for 60 s, and these total lipid extracts (TLEs) were then stored at -80°C until analysis. Before analysis, the samples were diluted 100 times in MeOH/IPA/H2O 65:35:5 (v/v/v) before injection.

The samples were separated on an HTC PAL system (CTC Analytics AG) coupled with a Transcend 1250 liquid chromatographic system (Thermo Fisher Scientific, Inc.) using a Kinetex C8 2.6 µm 2.1 × 150 mm column (Phenomenex, Sydney, NSW, Australia). High-resolution mass spectrometry using a Q-Exactive mass spectrometer (Thermo Fisher Scientific, Inc.) was performed as previously described (3). The relative amount of each lipid was semi-quantified as the area of its corresponding chromatographic peak.

### Quantitative real-time PCR

Total RNA was extracted using the RNeasy Kit (Qiagen). (2). Briefly, total RNA was extracted from mouse and human tissues using RNeasy Kit (Qiagen, Hilden, Germany). To quantify mRNA levels, 1µg of RNA was transcribed into cDNA using Superscript IV reverse transcription reagents (Invitrogen, Thermo Fisher Scientific Inc.) in a final volume of 25 µL. Dilutions ranging from 1/75 to 1/5 of cDNA were used to measure mRNA. The expression of the genes of interest was analyzed by RT-PCR using TaqMan^{®} Gene Expression Assays (Thermo Fisher Scientific Inc.) and standardized TaqMan^{®} probes on a LightCycler^{®} 480 Real-Time PCR System (Roche Diagnostics GmbH). Relative quantification was carried out using the 'Delta-Delta Ct' (ΔΔCt) method with human RPLP0 as an endogenous control. Transcripts were quantified in triplicate for each sample.

### Statistical analysis

For lipidomics data processing including filtering and normalization, we used MetaboAnalystR package (*4*). Then, differentially intensities between groups were detected using the Limma package (5). A major benefit of the Limma procedure is that it allows for factorial analysis in the specification of the linear model. As such, we were able to adjust for age between phenotypes by including two-levels, children and adult in the specification of the Age factor. Therefore, for ccALD and AMN or cAMN differences, the design analysis specified only one contrast [(ALD.child-CTL.child) - (ALD.adult-CTL.adult)], characterizing the genotype-by-age interaction between X-ALD and controls in children and adults.

Relative gene expression was examined for normality with the Shapiro-Wilk test. For normal distributed data significant differences were determined using 2-factor analysis in the specification of the linear model and pairwise comparisons were performed using t-test. Otherwise, Scheirer-Ray-Hare (SRH) test followed by Wilcoxon's post hoc test were used for non-normal distributed data. Multiple comparisons were adjusted using Benjamini & Hochberg test.

### Enrichment

To evaluate which class lipids were enriched, we computed Fisher's exact test and Monte Carlo simulated chi-square test.

### LMM

For ILs, oxylipids and cholic acid levels a linear mixed model was computed with age as a covariate and a random patient ID factor to account for patient replications using "nlme" R package (https://link.springer.com/book/10.1007/b98882). For pairwise comparisons estimated marginal means (EMMs) were performed adjusted with Bonferroni's method using "emmeans" R package (https://CRAN.R-project.org/package=emmeans).

### Penalty regression models

All penalty regression methods in this study were conducted using the "glmnet" package in the statistical software R (6). The 'cv.glmnet' function incorporates a number of model fitting practices including n-fold cross validation and Least Absolute Shrinkage and Selection Operator (LASSO) regularization to reduce model overfitting. The Lasso penalty parameter was selected using the automatic cross-validation. By default, our method uses leave-one-study-out crossing validation, where n in n-fold corresponds to the number of samples in our experiment.

### Multivariate Analysis representation

Principal Component Analysis (PCA) and Partial Least-Squares Discriminant Analysis (PLS-DA) for lipidomics data were computed using MetaboAnalystR package.

*Receiver Operating Characteristic* The power to discriminate ccALD and AMN or cAMN using lipidomics was evaluated by calculating the area under the curve (AUC) of the receiver operating characteristic (ROC) curve. The pROC package in R was used to calculate AUCs along with their standard errors and 95% confidence intervals (7). The DeLong test was used to compare the areas under the correlated ALD.Child-ALD.Adult and CTL.Child-CTL.Adult ROC curves.

### RESULTS

### GD1 (d18:1/20:0), SM (d18:2/22:1) and LPC-26:1 are able to discriminate among X-ALD phenotypes

Lipidomic analysis using mass spectrometry was used to characterize the lipid profile in plasma from ccALD (n = 13) and AMN (n = 15) patients, and healthy age-matched controls (adult and child controls, CTL, n = 15/15). After lipid extraction, we performed non-targeted lipidomic analyses by RP-LC/HRMS and detected 40883 molecules from plasma with 642 annotated unique lipids from different lipid classes: free fatty acids (FFA), acylcarnitines (AC), cholesteryl esters (CE), diacylglycerols (DAG), triacylglycerols (TAG), lysophosphatidylcholines (LPC), lysophosphatidylethanolamines (LPE), lysophosphatidylinositols (LPI), lyso-phosphatidylserines (LPS), phosphatidylcholines, (PC), phosphatidylethanolamines (PE), phosphatidylglycerols (PG), phosphatidylinositols (PI), phosphatidylserines (PS), ceramides (Cer), hexosylceramides (HexCer), dihexosylceramides (DiHexCer), lactoceramides (LacCer), sphingomyelins (SM), sulfoglycosphingolipids (Sulfatides, ST), and gangliosides (GA). Tridimensional PCA (an unsupervised technique) and PLS-DA (a supervised technique) (*1*) of the 642 lipidic species revealed ccALD- and AMN-specific sample distribution.

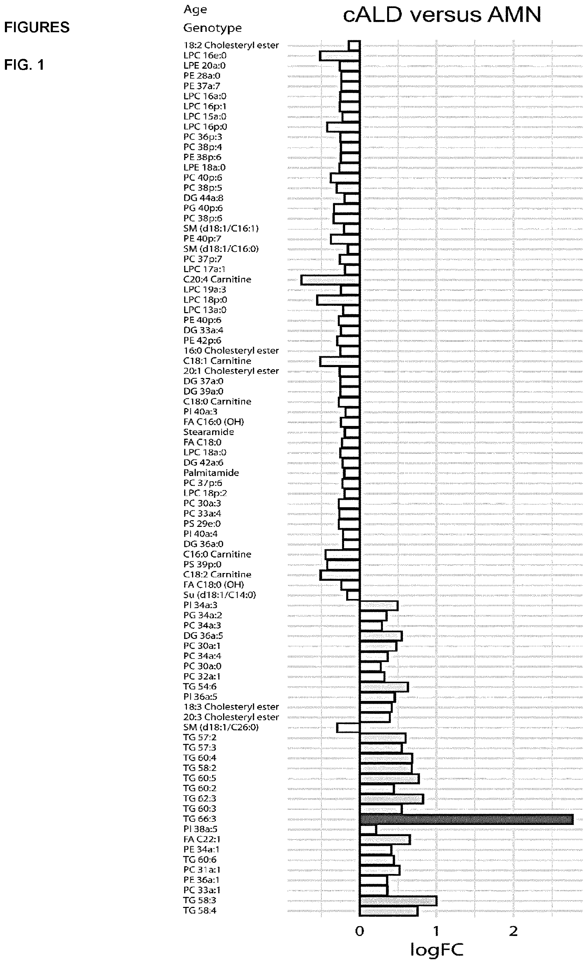

To further explore the lipidic differences between control, ccALD, and AMN individuals, we performed a two-factor statistical analysis to adjust the age effect, resulting with 66 molecules significantly differential between AMN and ccALD patients (Fig. 1), 13 TAG of these were accumulated in ccALD compared to AMN. The class lipid enrichment of these 66 lipid species showed a significant higher representation of LPC and AC acylcarnitines (Table 1):

**Table 1**

| Product Name | Class | logFC | adj.P.Val | AUC(%) | DeLong's test |
|---|---|---|---|---|---|
| LPC 16e:0 | LPC | -1.5744 | 0.0020 | 80.5 | **1.3E-05** |
| LPC 16p:0 | LPC | -1.3100 | 0.0020 | 86.2 | **3.7E-05** |
| LPC 18p:0 | LPC | -1.6924 | 0.0020 | 74.4 | **1.2E-05** |
| PI 34a:3 | PI | 1.7866 | 0.0042 | 89.7 | **6.7E-06** |
| PG 40p:6 | PG | -1.2442 | 0.0072 | 90.8 | **1.4E-06** |
| PS 39p:0 | PS | -1.4654 | 0.0072 | 65.6 | **2.6E-04** |
| PE 40p:7 | PE | -1.3500 | 0.0074 | 83.1 | **1.3E-05** |
| DG 36a:0 | DAG | -0.7515 | 0.0074 | 75.9 | **2.0E-06** |
| PC 30a:3 | PC | -0.8908 | 0.0088 | 62.6 | **4.4E-05** |
| TG 58:3 | TAG | 3.1660 | 0.0088 | 83.6 | **3.9E-03** |
| TG 60:5 | TAG | 2.5188 | 0.0088 | 83.6 | **8.9E-05** |
| PC 31a:1 | PC | 1.7675 | 0.0105 | 85.6 | **2.4E-03** |
| PS 29e:0 | PS | -0.8964 | 0.0105 | 73.3 | **1.6E-04** |
| 16:0 Cholesteryl ester | CE | -0.8810 | 0.0114 | 73.8 | **2.6E-04** |
| DG 36a:5 | DAG | 1.5965 | 0.0115 | 76.9 | **2.7E-04** |
| PC 40p:6 | PC | -1.3472 | 0.0119 | 89.7 | **2.3E-04** |
| C20:4 Carnitine | AC | -2.2128 | 0.0120 | 75.4 | **2.1E-03** |
| PC 38p:6 | PC | -1.1984 | 0.0130 | 90.8 | **3.5E-04** |
| 20:1 Cholesteryl ester | CE | -0.8872 | 0.0131 | 76.9 | **2.0E-04** |
| PC 33a:4 | PC | -0.8630 | 0.0155 | 74.9 | **1.2E-02** |
| TG 60:4 | TAG | 2.1668 | 0.0155 | 86.2 | **1.2E-04** |
| 20:3 Cholesteryl ester | CE | 1.3194 | 0.0167 | 66.2 | **1.3E-03** |
| LPC 16p:1 | LPC | -0.7793 | 0.0169 | 82.6 | **8.0E-04** |
| LPE 18a:0 | LPE | -0.7927 | 0.0169 | 90.8 | **1.7E-04** |
| LPC 16a:0 | LPC | -0.7647 | 0.0175 | 81 | **1.4E-03** |
| PC 38p:5 | PC | -1.0845 | 0.0175 | 88.7 | **3.5E-04** |
| PE 34a:1 | PE | 1.4242 | 0.0175 | 81.5 | **6.4E-03** |
| PE 40p:6 | PE | -0.9454 | 0.0175 | 75.9 | **2.0E-04** |
| 18:3 Cholesteryl ester | CE | 1.3848 | 0.0175 | 64.6 | **2.0E-03** |
| C16:0 Carnitine | AC | -1.2459 | 0.0175 | 61 | **1.1E-02** |
| PI 40a:4 | PI | -0.7073 | 0.0176 | 62 | **4.3E-03** |
| DG 33a:4 | DAG | -0.8683 | 0.0176 | 70.8 | **4.8E-04** |
| TG 58:4 | TAG | 2.3015 | 0.0176 | 81 | **4.3E-03** |
| 18:2 Cholesteryl ester | CE | -0.4939 | 0.0202 | 79 | **1.1E-04** |
| PI 38a:5 | PI | 0.7884 | 0.0205 | 85.1 | **1.3E-02** |
| PC 34a:3 | PC | 0.8962 | 0.0217 | 74.9 | **2.3E-03** |
| TG 54:6 | TAG | 2.0031 | 0.0217 | 66.2 | **4.8E-03** |
| PG 34a:2 | PG | 1.1827 | 0.0230 | 73.3 | **2.9E-03** |
| PC 33a:1 | PC | 1.1082 | 0.0231 | 85.1 | **1.2E-03** |
| PE 38p:6 | PE | -0.9295 | 0.0231 | 80 | **1.2E-03** |
| C18:1 Carnitine | AC | -1.4927 | 0.0260 | 66.2 | **3.7E-03** |
| DG 42a:6 | DAG | -0.7557 | 0.0268 | 61 | **8.9E-04** |
| TG 58:2 | TAG | 2.1600 | 0.0273 | 78.5 | **4.6E-03** |
| SM (d18:1/C16:1) | SM | -0.7077 | 0.0273 | 91.3 | **1.5E-03** |
| LPC 13a:0 | LPC | -0.7847 | 0.0283 | 72.8 | **2.7E-03** |
| LPC 18a:0 | LPC | -0.7519 | 0.0299 | 68.2 | **2.7E-03** |
| PI 36a:5 | PI | 1.5859 | 0.0299 | 70.8 | **2.6E-03** |
| TG 62:3 | TAG | 2.6257 | 0.0323 | 69.2 | **1.2E-02** |
| PC 32a:1 | PC | 1.1010 | 0.0355 | 63.1 | **1.9E-03** |
| PC 37p:7 | PC | -0.9609 | 0.0355 | 77.4 | **3.6E-03** |
| LPC 19a:3 | LPC | -0.7109 | 0.0386 | 73.8 | **3.1E-03** |
| LPE 20a:0 | LPE | -0.7541 | 0.0386 | 64.6 | **3.2E-03** |
| PC 37p:6 | PC | -0.7946 | 0.0386 | 70.8 | **1.3E-03** |
| C18:2 Carnitine | AC | -1.4794 | 0.0386 | 59.5 | **2.6E-02** |
| PE 28a:0 | PE | -0.8210 | 0.0387 | 71.3 | **3.7E-03** |
| PI 40a:3 | PI | -0.6281 | 0.0404 | 56.4 | **2.0E-02** |
| PC 30a:1 | PC | 1.5905 | 0.0421 | 65.6 | **4.8E-03** |
| PE 36a:1 | PE | 1.1618 | 0.0421 | 83.1 | **1.4E-02** |
| TG 66:3 | TAG | 8.6472 | 0.0421 | 73.3 | **9.2E-02** |
| FA C22:1 | FFA | 2.2620 | 0.0433 | 83.1 | 1.1E-01 |
| PC 38p:4 | PC | -0.8439 | 0.0433 | 77.9 | **3.3E-03** |
| PE 37a:7 | PE | -0.8124 | 0.0433 | 70.3 | **6.5E-03** |
| TG 60:3 | TAG | 1.7071 | 0.0453 | 79.5 | **2.8E-03** |
| PC 34a:4 | PC | 1.0830 | 0.0485 | 65.6 | **1.9E-02** |
| FA C16:0 (OH) | FFA | -0.7612 | 0.0485 | 61.5 | **6.5E-03** |
| FA C18:0 (OH) | FFA | -0.7362 | 0.0485 | 64.5 | **3.9E-03** |

Significantly differential lipids identified in ccALD with respect to AMN in plasma of X-ALD patients (n = 15 AMN, n = 13 ccALD, n = 15 adult controls and n = 15 child controls). We performed two-factor analysis to adjust age effect between XALD phenotypes by including two-levels, children and adult in the specification of the Age factor. The AUC (Area under Curve) was calculated using the lipidomics intensities of the four groups. To identify the discriminatory capacity due solely to the difference in phenotypes and not to the difference in age, we applied the DeLong's test, which statistically evaluates the difference between the AUC of ccALD-AMN phenotypes and the AUC between child-adult controls. Cholesteryl esters (CE), ceramides (Cer), diacylglycerols (DAG), dihexosylceramides (DiHexCer), free fatty acids (FFA), fatty acylcarnitines (AC), hexosylceramides (HexCer), lysophosphatidylcholines (LPC), lysophosphatidylethanolamines (LPE), phosphatidylcholines, (PC), phosphatidylethanolamines (PE), phosphatidylglycerols (PG), phosphatidylinositols (PI), phosphatidylserines (PS), sphingosines (SPH), and sphingomyelins (SM), and triacylglycerols (TAG).

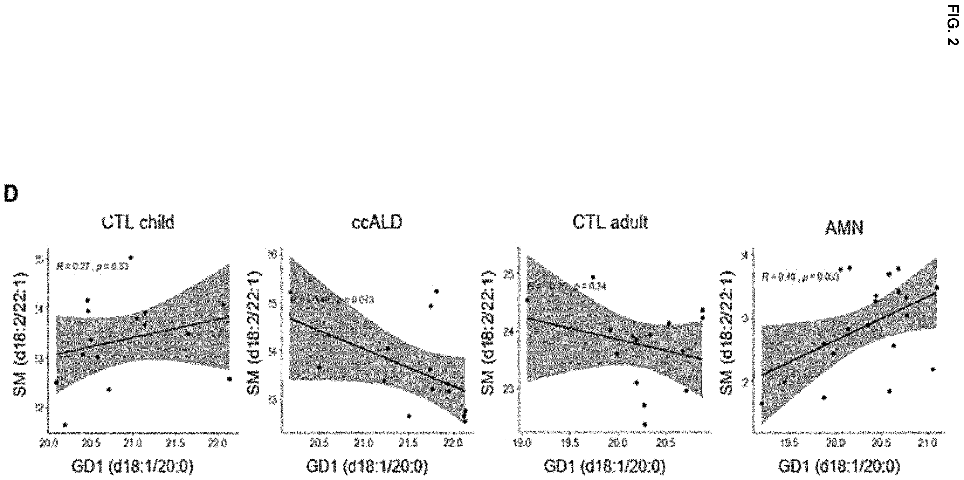

A penalized logistic regression model was also built in order to discriminate between X-ALD phenotypes and control subjects. The best model was the combination of three different species: GD1 (d18:1/20:0), SM (d18:2/22:1) and LPC 26:1. The model correctly classified ccALD (100%), AMN (100%), and CTL (100%) phenotypes. We observed a positive and an inverse correlation between the GD1 (d18:1/20:0) and the SM (d18:2/22:1) levels in AMN, and in ccALD patients, respectively. These correlations are X-ALD-specific and independent of age, as in age-matched controls these associations are not present (Fig. 2).

PCA-Biplot graph showed a differential sample distribution for control, AMN and ccALD subjects in this lipidomic study, demonstrating that they can be stratified into subgroups according to these three lipid species.

Lipidomic analysis also showed that some species of sphingolipids, most notably GM3 and GD1, are higher in plasma from ccALD compared to AMN patients or age-matched healthy individuals. DAG, and especially TAG with long-chain FA also presented a large number of species higher in plasma from ccALD compared to AMN patients.

### Galactosylceramide levels are reduced in normal appearing brain white matter ccALD patients

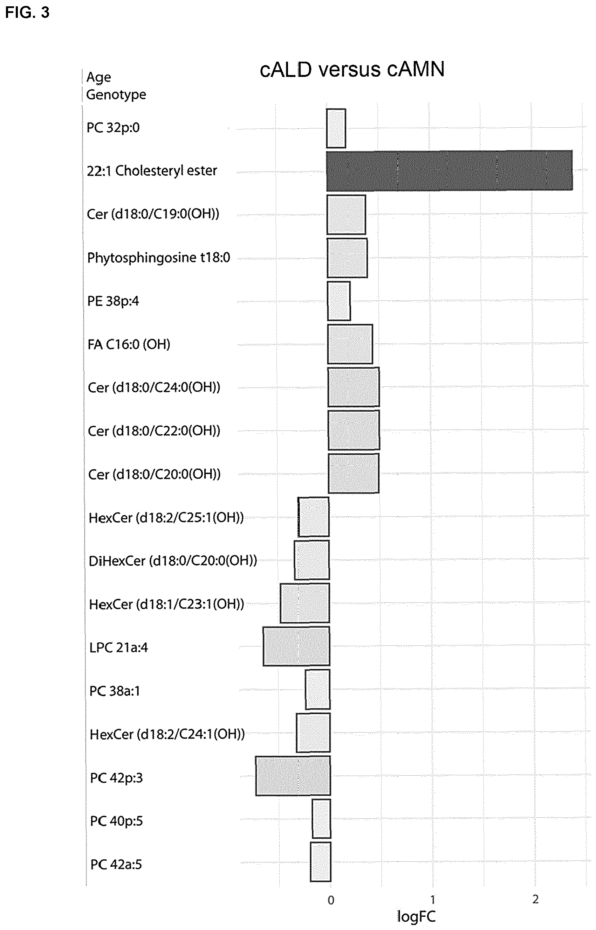

Lipidomic analysis using mass spectrometry was also used to characterize the lipid profile in normal-appearing brain white matter (NAWM) from ccALD (n = 9), and cAMN (n = 9) patients, and healthy age-matched controls (adult and child controls, n = 12/12). After lipid extraction, we performed non-targeted lipidomic analyses by RP-LC/HRMS and detected 60847 molecules, with 834 annotated unique lipids from different lipid classes. To further explore the lipidic differences between control, ccALD, and cAMN individuals, a multivariate statistical analysis was employed, including PCA (an unsupervised technique) and PLS-DA (a supervised technique) (1). Both techniques uncovered ccALD- and cAMN-specific sample distribution. The factor analysis adjusted by age of X-ALD and control brain samples revealed that 18 molecules were significantly different between cAMN and ccALD patients (Fig. 3 and Table 2), highlighting free saturated hydroxylated fatty acids (OH-FA) or complex lipids containing hydroxylated fatty acids.

It was also found that dihydroceramides containing OH-FA (DhCer(OH)) were elevated in ccALD patients while both OH-FA-hexosylceramides (HexCer(OH)), and OH-FA-hexosyldihydroceramides (HexDhCer(OH)) were reduced in ccALD compared to cAMN and age-matched healthy individuals. However, the large majority of HexCer(OH) in brain white matter is GalCer (2), which is precisely the main sphingolipid species in myelin. We thus infer that hydroxylated galactosylceramides (GalCer(OH)) were reduced in ccALD patients.

The results are summarized in Table 2 which provides the significantly differential lipids identified in ccALD with respect to cAMN in non-affected white brain samples of X-ALD patients (n = 9 cAMN, n = 9 ccALD, n = 12 adult controls and n = 12 child controls).

**Table 2**

| Product Name | Class | logFC | P.Value | AUC(%) | DeLong's test |
|---|---|---|---|---|---|
| Cer (d18:0/C22:0(OH)) | Cer | 0.4999 | 0.0046 | 76.5 | **8.50E-03** |
| Cer (d18:0/C20:0(OH)) | Cer | 0.4918 | 0.0051 | 75.3 | **1.38E-02** |
| Cer (d18:0/C24:0(OH)) | Cer | 0.5005 | 0.0067 | 75.3 | **2.13E-02** |
| FA C16:0 (OH) | FFA | 0.4369 | 0.0132 | 70.4 | 5.40E-02 |
| PC 42p:3 | PC | -0.7272 | 0.0244 | 81.5 | **1.65E-02** |
| PC 32p:0 | PC | 0.1841 | 0.0273 | 87.7 | **5.48E-03** |
| LPC 21a:4 | LPC | -0.6467 | 0.0301 | 75.3 | 7.14E-02 |
| HexCer (d18:2/C25:1(OH)) | HexCer | -0.2958 | 0.0322 | 93.8 | **2.00E-02** |
| Cer (d18:0/C19:0(OH)) | Cer | 0.3743 | 0.0328 | 70.4 | 5.25E-02 |
| 22:1 Cholesteryl ester | CE | 2.3961 | 0.0336 | 80.2 | **1.09E-02** |
| PC 38a:1 | PC | -0.2390 | 0.0339 | 75.3 | 7.24E-02 |
| PC 40p:5 | PC | -0.1779 | 0.0380 | 70.4 | **2.71E-02** |
| PE 38p:4 | PE | 0.2181 | 0.0382 | 86.4 | **3.95E-03** |
| DiHexCer (d18:0/C20:0(OH)) | DiHexCer | -0.3346 | 0.0387 | 97.5 | **1.93E-02** |
| HexCer (d18:1/C23:1(OH)) | HexCer | -0.4750 | 0.0428 | 90.1 | **1.09E-02** |
| HexCer (d18:2/C24:1(OH)) | HexCer | -0.3230 | 0.0432 | 87.7 | **1.90E-02** |
| PC 42a:5 | PC | -0.1985 | 0.0440 | 66.7 | **3.75E-02** |
| Sphingosine d18:1 | SPH | 0.3884 | 0.0479 | 63 | 2.03E-01 |

Significantly differential lipids identified in ccALD with respect to cAMN in non-affected white brain samples of X-ALD patients (n = 9 cAMN, n = 9 ccALD, n = 12 adult controls and n = 12 child controls). We performed two-factor analysis to adjust age effect between XALD phenotypes by including two-levels, children and adult in the specification of the Age factor. The AUC (Area under Curve) was calculated using the lipidomics intensities of the four groups. To identify the discriminatory capacity due solely to the difference in phenotypes and not to the difference in age, we applied the DeLong's test, which statistically evaluates the difference between the AUC of ccALD-AMN phenotypes and the AUC between child-adult controls. Cholesteryl esters (CE), ceramides (Cer), diacylglycerols (DAG), dihexosylceramides (DiHexCer), free fatty acids (FFA), fatty acylcarnitines (AC), hexosylceramides (HexCer), lysophosphatidylcholines (LPC), lysophosphatidylethanolamines (LPE), phosphatidylcholines, (PC), phosphatidylethanolamines (PE), phosphatidylglycerols (PG), phosphatidylinositols (PI), phosphatidylserines (PS), sphingosines (SPH), and sphingomyelins (SM), and triacylglycerols (TAG).

### Oxylipid levels are increased in plasma from cerebral X-ALD but not in AMN patients

We have quantified several oxylipids (5-HETE, 9-HETE, 11-HETE, 12-HETE, 15-HETE, 5,6-DHET, 8,9-DHET, 11,12-DHET, 4-HDHA, 13-HDHA, 17-HDHA, 15-HETrE) and free eicosapentaenoic acid (EPA) levels in plasma samples of healthy controls (n = 46), AMN (n = 47), and cerebral X-ALD patients (ccALD and cAMN patients, n = 27). We observed that all measured oxylipids were raised in plasma from cALD patients except for EPA, which was only accumulated in AMN patients.

### Cholic acid level is decreased in plasma from cerebral X-ALD patients

We quantified cholic acid in plasma from AMN (n = 47) and cALD patients (n = 27), and age-matched healthy controls (n = 46). We found that cholic acid was decreased in cerebral X-ALD patients compared with AMN patients and healthy controls (Table 4) .

### REFERENCES

1. J. Trygg, E. Holmes, T. Lundstedt, Chemometrics in metabonomics. J. Proteome Res. 6, 469-479 (2007).
2. H. Hama, Fatty acid 2-Hydroxylation in mammalian sphingolipid biology. Biochim. Biophys. Acta 1801, 405-414 (2010).
3. D. C. Pant, S. Aguilera-Albesa, A. Pujol, Ceramide signalling in inherited and multifactorial brain metabolic diseases. Neurobiol. Dis. 143, 105014 (2020).
4. Z. Pang, J. Chong, S. Li, J. Xia, MetaboAnalystR 3.0: Toward an Optimized Workflow for Global Metabolomics. Metabolites 10, (2020).
5. M. E. Ritchie et al., limma powers differential expression analyses for RNA-sequencing and microarray studies. Nucleic Acids Res. 43, e47 (2015).
6. J. Friedman, T. Hastie, R. Tibshirani, Regularization Paths for Generalized Linear Models via Coordinate Descent. J Stat Softw 33, 1-22 (2010).
7. X. Robin et al., pROC: an open-source package for R and S+ to analyze and compare ROC curves. BMC Bioinformatics 12, 77 (2011).

### Clauses

For reasons of completeness, various aspects of the invention are set out in the following numbered clauses:
**Clause 1.** An *in vitro* method for classifying a subject suffering from an X-linked adrenoleukodystrophy as suffering from the cerebral inflammatory adrenoleukodystrophy (cALD) phenotype or as suffering from the adrenomyeloneuropathy (AMN) phenotype, the method comprising:
   (a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
   (b) obtaining a reference value for each one of the lipidic components in a population already identified as having cALD phenotype or an AMN phenotype;
   (c) comparing each one of the quantified lipidic components determined in step (a) with the corresponding reference value; and
   (d) identifying a deviation of the lipid level in the test sample with respect to the cALD or AMN reference value.
**Clause 2.** The *in vitro* method of clause 1, wherein the reference value for each one of the lipids is obtained from a population of subjects with an AMN phenotype, and:
   the subject will have cALD phenotype when the isolated test sample comprises one or more of the markers of Table 3 at a level above the corresponding reference value(s); and/or one or more markers of Table 4 below the corresponding reference value(s); and
   the subject will have an AMN phenotype when the isolated test sample comprises one or more of the markers of Table 3 at a level equal or lower than the corresponding reference value(s); and/or one or more markers of Table 4 above the corresponding reference value(s).
**Clause 3**. The *in vitro* method of any one of the clauses 1-2, wherein step (b) further comprises determining the level of each one of the lipids in a population of samples from healthy people, both adults and children, and step (c) is performed by a two-factor statistical analysis.
**Clause 4.** The *in vitro* method of any one of the clauses 1-3, wherein step (b) comprises determining the level of one or more of GD1, SM, LPC 26:0, LPC 26:1, and GM3.
**Clause 5.** The *in vitro* method of clause 4, wherein it is determined the level of GD1, SM and LPC 26:1; or alternatively of GD1, SM, and LPC 26:0.
**Clause 6.** An *in vitro* method of diagnosis cALD, the method comprising:
   (a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
   (b) obtaining a reference value for each one of the lipidic components in a population already identified as having an AMN phenotype;
   (c) comparing each one of the quantified lipidic components determined in step (a) with the corresponding reference value; and
   (d) identifying a deviation of the lipid level from the test sample with respect to the AMN reference value,
   wherein the subject will suffer from cALD phenotype when the isolated test sample comprises one or more of the markers of Table 3 at a level above the corresponding reference value(s); and/or one or more markers of Table 4 below the corresponding reference value(s).
**Clause 7.** An *in vitro* method of diagnosis or prognosis of AMN, the method comprising:
   (a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
   (b) obtaining a reference value for each one of the lipidic components in a population already identified as having an AMN phenotype;
   (c) comparing each one of the quantified lipidic components determined in step (a) with the corresponding reference value; and
   (d) identifying a deviation of the lipid level from the test sample with respect to the AMN reference value,
   wherein the subject will have an AMN phenotype when the isolated test sample comprises one or more of the markers of Table 3 or the cholic acid is at a level equal or lower than the corresponding reference value(s); and/or one or more markers of Table 4 above the corresponding reference value(s).
**Clause 8.** The *in vitro* method of clause 7, which is a prognostic method, wherein when one or more of the following oxylipids: 11-HETE, 11,12-DHET, 15-oxoETE, 17-HDHA, 5-HETE, 5,6-DHET, 6R-LXA4, 8,9-DHET, and 9-HETE, is higher than the corresponding reference value(s), this will be indicative of bad prognosis; and
   when cholic acid is lower than the reference value, this will be indicative of bad prognosis.
**Clause 9.** The *in vitro* method of clause 8, wherein the bad prognosis is the evolution to a cerebral inflammatory form.
**Clause 10.** An *in vitro* method for determining the response of a treatment in a subject suffering from cALD, the method comprising:
   (a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3; in two samples of the subject, one of them taken before and the other taken after starting the therapy; or, alternatively, each one of them taken in two separate moments after starting the therapy; and
   (b) comparing the quantified lipidic components in the two moments of time;
   wherein
   when the level of one or more of the markers of Table 3 is reduced; and/or the level of one or more markers of Table 4 is increased, it will be indicative that the subject positively responds to the treatment.
**Clause 11.** An *in vitro* method for determining the response of a treatment in a subject suffering from AMN, the method comprising:
   (a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3; in two samples of the subject, one of them taken before and the other taken after starting the therapy; or, alternatively, each one of them taken in two separate moments after starting the therapy; and
   (b) comparing the quantified lipidic components in the two moments of time;
   wherein
   when the level of one or more of the markers of Table 3 is increased; and/or the level of one or more markers of Table 4 is decreased, it is indicative that the subject positively responds to the treatment.
**Clause 12.** The *in vitro* method of any one of the preceding clauses, wherein the isolated test sample is a biological fluid sample.
**Clause 13.** The *in vitro* method of any one of the preceding clauses, wherein the biological fluid sample is plasma, serum, whole blood or cerebrospinal fluid.
**Clause 14.** The *in vitro* method of any one of the preceding clauses 1, 4-13, wherein it is determined one or more of the following:
   - the combination of GD1, SM and LPC 26:1;
   - oxylipids listed in Table 3; and
   - cholic acid;
   in an isolated plasma sample of the subject.
**Clause 15.** The *in vitro* method of any one of the preceding clauses 1, 4-13, wherein it is determined the level of one or more galactosylceramides in an isolated cerebrospinal fluid sample of the subject.
**Clause 16.** The *in vitro* method of any one of the preceding clauses, wherein it is further determined the level of one or more of IL-18, IL-1RA or FA2H or UGT8 expression.
**Clause 17.** An *in vitro* diagnostic method of cALD in a subject, the method comprising:
   - determining the expression level of one or more of the following enzymes: ceramide synthase (CerS) 1, 4, 6, GAL3ST1, FA2H, or the UGT-8, and
   - comparing with the corresponding reference value from healthy children population;
   wherein when the gene expression activity is lower than the reference value, this will indicate that the subject suffers from cALD.
**Clause 18.** A screening method for identifying candidate compounds suitable for the treatment of cALD, the method comprising contacting the candidate compound with a medium comprising one of the following enzymes: ceramide synthase (CerS) 1, 4, 6, GAL3ST1, FA2H or the UGT-8, and determining the enzymatic activity, provided that when the compound increases the gene expression activity, this will mean that the tested compound is a candidate for the treatment of cALD.
**Clause 19.** The method of any one of the preceding clauses 1-6, 10, and 12-18, wherein the subject is a child.

## Claims

1. An *in vitro* method for classifying a subject suffering from an X-linked adrenoleukodystrophy as suffering from the cerebral inflammatory adrenoleukodystrophy (cALD) phenotype or as suffering from the adrenomyeloneuropathy (AMN) phenotype, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
(b) obtaining a reference value for each one of the lipidic components in a population already identified as having cALD phenotype or an AMN phenotype;
(c) comparing each one of the quantified lipidic components determined in step (a) with the corresponding reference value; and
(d) identifying a deviation of the lipid level in the test sample with respect to the cALD or AMN reference value.

2. The *in vitro* method of claim 1, wherein the reference value for each one of the lipids is obtained from a population of subjects with an AMN phenotype, and:
the subject will have cALD phenotype when the isolated test sample comprises one or more of the markers of Table 3 at a level above the corresponding reference value(s); and/or one or more markers of Table 4 below the corresponding reference value(s); and
the subject will have an AMN phenotype when the isolated test sample comprises one or more of the markers of Table 3 at a level equal or lower than the corresponding reference value(s); and/or one or more markers of Table 4 above the corresponding reference value(s).

3. The *in vitro* method of any one of the claims 1-2, wherein step (b) further comprises determining the level of each one of the lipids in a population of samples from healthy people, both adults and children, and step (c) is performed by a two-factor statistical analysis.

4. The *in vitro* method of any one of the claims 1-3, wherein step (b) comprises determining the level of one or more of GD1, SM, LPC 26:0, LPC 26:1, and GM3.

5. The *in vitro* method of claim 4, wherein it is determined the level of GD1, SM and LPC 26:1; or alternatively of GD1, SM, and LPC 26:0.

6. An *in vitro* method of diagnosis cALD, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
(b) obtaining a reference value for each one of the lipidic components in a population already identified as having an AMN phenotype;
(c) comparing each one of the quantified lipidic components determined in step (a) with the corresponding reference value; and
(d) identifying a deviation of the lipid level from the test sample with respect to the AMN reference value,
wherein the subject will suffer from cALD phenotype when the isolated test sample comprises one or more of the markers of Table 3 at a level above the corresponding reference value(s); and/or one or more markers of Table 4 below the corresponding reference value(s).

7. An *in vitro* method of diagnosis or prognosis of AMN, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3;
(b) obtaining a reference value for each one of the lipidic components in a population already identified as having an AMN phenotype;
(c) comparing each one of the quantified lipidic components determined in step (a) with the corresponding reference value; and
(d) identifying a deviation of the lipid level from the test sample with respect to the AMN reference value,
wherein the subject will have an AMN phenotype when the isolated test sample comprises one or more of the markers of Table 3 or the cholic acid, at a level equal or lower than the corresponding reference value(s); and/or one or more markers of Table 4 above the corresponding reference value(s).

8. The *in vitro* method of claim 7, which is a prognostic method, wherein:
when one or more of the oxylipids: 11-HETE, 11,12-DHET, 15-oxoETE, 17-HDHA, 5-HETE, 5,6-DHET, 6R-LXA4, 8,9-DHET, and 9-HETE, is higher than the corresponding reference value(s), this will be indicative of bad prognosis; and
when cholic acid is lower than the reference value, this will be indicative of bad prognosis.

9. The *in vitro* method of claim 8, wherein the bad prognosis is the evolution to a cerebral inflammatory form.

10. An *in vitro* method for determining the response of a treatment in a subject suffering from cALD, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3; in two samples of the subject, one of them taken before and the other taken after starting the therapy; or, alternatively, each one of them taken in two separate moments after starting the therapy; and
(b) comparing the quantified lipidic components in the two moments of time;
wherein
when the level of one or more of the markers of Table 3 is reduced; and/or the level of one or more markers of Table 4 is increased, it will be indicative that the subject positively responds to the treatment.

11. An *in vitro* method for determining the response of a treatment in a subject suffering from AMN, the method comprising:
(a) performing a lipidomic analysis of the isolated test sample to quantify one or more of the following markers: 11,12-DHET, 11-HETE, 15-oxoETE, 16:0 Cholesteryl ester, 17-HDHA, 18:2 Cholesteryl ester, 18:3 Cholesteryl ester, 20:1 Cholesteryl ester, 20:3 Cholesteryl ester, 22:1 Cholesteryl ester, 5,6-DHET, 5-HETE, 6R-LXA4, 8,9-DHET, 9-HETE, C16:0 Carnitine, C18:1 Carnitine, C18:2 Carnitine, C20:4 Carnitine, Cer (d18:0/C19:0(OH)), Cer (d18:0/C20:0(OH)), Cer (d18:0/C22:0(OH)), Cer (d18:0/C24:0(OH)), Cholic acid, DG 33a:4, DG 36a:0, DG 36a:5, DG 42a:6, DiHexCer (d18:0/C20:0(OH)), FA C16:0 (OH), FA C16:0 (OH), FA C18:0 (OH), FA C22:1, HexCer (d18:1/C23:1(OH)), HexCer (d18:2/C24:1(OH)), HexCer (d18:2/C25:1(OH)), LPC 13a:0, LPC 16a:0, LPC 16e:0, LPC 16p:0, LPC 16p:1, LPC 18a:0, LPC 18p:0, LPC 19a:3, LPC 21a:4, LPE 18a:0, LPE 20a:0, PC 30a:1, PC 30a:3, PC 31a:1, PC 31a:1, PC 32a:1, PC 32p:0, PC 33a:1, PC 33a:4, PC 33a:4, PC 34a:3, PC 34a:4, PC 37p:6, PC 37p:7, PC 38a:1, PC 38p:4, PC 38p:5, PC 38p:6, PC 40p:5, PC 40p:6, PC 42a:5, PC 42p:3, PE 28a:0, PE 34a:1, PE 36a:1, PE 37a:7, PE 38p:4, PE 38p:6, PE 40p:6, PE 40p:7, PG 34a:2, PG 40p:6, PI 34a:3, PI 36a:5, PI 38a:5, PI 40a:3, PI 40a:4, PS 29e:0, PS 39p:0, SM (d18:1/C16:1), Sphingosine d18:1, TG 54:6, TG 58:2, TG 58:3, TG 58:4, TG 60:3, TG 60:4, TG 60:5, TG 62:3, and TG 66:3; in two samples of the subject, one of them taken before and the other taken after starting the therapy; or, alternatively, each one of them taken in two separate moments after starting the therapy; and
(b) comparing the quantified lipidic components in the two moments of time;
wherein
when the level of one or more of the markers of Table 3 is increased; and/or the level of one or more markers of Table 4 is decreased, it is indicative that the subject positively responds to the treatment.

12. The *in vitro* method of any one of the preceding claims, wherein the isolated test sample is a biological fluid sample.

13. The *in vitro* method of any one of the preceding claims, wherein the biological fluid sample is plasma, serum, whole blood or cerebrospinal fluid.

14. The *in vitro* method of any one of the preceding claims 1, 4-13, wherein it is determined one or more of the following:
- the combination of GD1, SM and LPC 26:1;
- oxylipids listed in Table 3; and
- cholic acid;
in an isolated plasma sample of the subject.

15. The *in vitro* method of any one of the preceding claims, wherein it is further determined the level of one or more of IL-18, IL-1RA or UGT8 expression.
